# EUROPEAN PATENT SPECIFICATION

(11) **EP 4 046 659 B1**
(45) Date of publication and mention of the grant of the patent: **06.05.2026**
(21) Application number: 21833229.4
(22) Date of filing: 30.03.2021
(51) Int. Cl.: A61K 49/22, A61K 41/00, A61K 31/337, A61P 35/00

(54) **MAGNETIC ULTRASOUND CONTRAST AGENT COMPOSITION, MAGNETIC ULTRASOUND CONTRAST AGENT, AND MAGNETIC MICROBUBBLE ULTRASOUND CONTRAST AGENT AND PREPARATION METHOD THEREFOR**
MAGNETISCHE ULTRASCHALLKONTRASTMITTELZUSAMMENSETZUNG, MAGNETISCHES ULTRASCHALLKONTRASTMITTEL UND MAGNETISCHES MIKROBLASEN-ULTRASCHALLKONTRASTMITTEL UND HERSTELLUNGSVERFAHREN DAFÜR
COMPOSITION D'AGENT DE CONTRASTE ULTRASONORE MAGNÉTIQUE, AGENT DE CONTRASTE ULTRASONORE MAGNÉTIQUE ET AGENT DE CONTRASTE ULTRASONORE À MICROBULLES MAGNÉTIQUES ET SON PROCÉDÉ DE PRÉPARATION

(30) Priority: 28.06.2020 CN 202010597816
(43) Date of publication of application: 24.08.2022
(73) Proprietor: Nanjing Leapsonics Technology Co., Ltd., Nanjing, Jiangsu 211800 (CN)
(72) Inventor: HUANG, Shuo, Nanjing, Jiangsu 211800 (CN); GUO, Wenyu, Nanjing, Jiangsu 211800 (CN); DONG, Feihong, Nanjing, Jiangsu 211800 (CN); ZHANG, Jue, Nanjing, Jiangsu 211800 (CN)
(74) Representative: Murgitroyd & Company
(86) International application number: PCT/CN2021/084076
(87) International publication number: WO 2022/001253

(56) References cited:
- CN-A- 101 711 870
- CN-A- 103 243 073
- CN-A- 104 622 848
- CN-A- 109 045 285
- CN-A- 110 575 551
- CN-A- 111 729 092
- US-A1- 2014 161 726
- CHING-HSIANG FAN ET AL: "Ultrasound/Magnetic Targeting with SPIO-DOX-Microbubble Complex for Image-Guided Drug Delivery in Brain Tumors", THERANOSTICS, vol. 6, no. 10, 18 June 2016 (2016-06-18), AU, pages 1542 - 1556, XP055756073, ISSN: 1838-7640, DOI: 10.7150/thno.15297
- HARDIANSYAH ANDRI ET AL: "Characterizations of doxorubicin-loaded PEGylated magnetic liposomes for cancer cells therapy", JOURNAL OF POLYMER RESEARCH, SPRINGER NETHERLANDS, DORDRECHT, vol. 26, no. 12, 25 November 2019 (2019-11-25), XP036932390, ISSN: 1022-9760, [retrieved on 20191125], DOI: 10.1007/S10965-019-1964-5
- BEGUIN ESTELLE ET AL: "Magnetic microbubble mediated chemo-sonodynamic therapy using a combined magnetic-acoustic device", JOURNAL OF CONTROLLED RELEASE, ELSEVIER, AMSTERDAM, NL, vol. 317, 13 November 2019 (2019-11-13), pages 23 - 33, XP086001781, ISSN: 0168-3659, [retrieved on 20191113], DOI: 10.1016/J.JCONREL.2019.11.013
- BEATA CHERTOK ET AL: "Circulating Magnetic Microbubbles for Localized Real-Time Control of Drug Delivery by Ultrasonography-Guided Magnetic Targeting and Ultrasound", THERANOSTICS, vol. 8, no. 2, 1 January 2018 (2018-01-01), AU, pages 341 - 357, XP055648869, ISSN: 1838-7640, DOI: 10.7150/thno.20781
- SONG WEIXIANG ET AL: "Magnetic nanobubbles with potential for targeted drug delivery and trimodal imaging in breast cancer: anin vitrostudy", vol. 12, no. 9, 13 March 2017 (2017-03-13), pages 991 - 1009, XP009533334, ISSN: 1743-5889, Retrieved from the Internet <URL:https://www.futuremedicine.com/doi/10.2217/nnm-2017-0027> [retrieved on 20170313], DOI: 10.2217/NNM-2017-0027
- PERRIER T ET AL: "Post-insertion into Lipid NanoCapsules (LNCs): From experimental aspects to mechanisms", INTERNATIONAL JOURNAL OF PHARMACEUTICS, ELSEVIER, NL, vol. 396, no. 1-2, 30 August 2010 (2010-08-30), pages 204 - 209, XP027169592, ISSN: 0378-5173, [retrieved on 20100619]
- SONG WEIXIANG, LUO YINDENG, ZHAO YAJING, LIU XINJIE, ZHAO JIANNONG, LUO JIE, ZHANG QUNXIA, RAN HAITAO, WANG ZHIGANG, GUO DAJING: "Magnetic nanobubbles with potential for targeted drug delivery and trimodal imaging in breast cancer: an in vitro study,", NANOMEDICINE, vol. 12, no. 9, 1 May 2017 (2017-05-01), pages 991 - 1009, XP009533334, ISSN: 1743-5889, DOI: 10.2217/nnm-2017-0027

## Description

### FIELD OF THE INVENTION

The invention relates to the field of biomedicine, in particular to a composition for magnetic ultrasound contrast agent, a magnetic ultrasound contrast agent including the composition for magnetic ultrasound contrast agent, a preparation method of a magnetic microbubble ultrasound contrast agent, and a magnetic microbubble ultrasound contrast agent prepared by the preparation method.

### BACKGROUND OF THE INVENTION

At present, efficient drugs for treating various cancers are usually insoluble in water, having great side effects, and prone to drug resistance. Study shows that target delivery may solve the above problem. In recent years, many researches have been made by scientists from all over the world in the field of nano-controlled release drug delivery system, such as a tumor-sensitive pH controlled release system, a drug-loaded nanoparticle. Although these nano-drug delivery systems may realize the responsive release of a drug at the tumor site, there are also some problems: such as insufficient of local release amount and low response sensitivity of tumor; especially in a high flow rate area, it is difficult to achieve drug-enriched delivery and visual controlled release.

In recent years, Ultrasonic Targeted Microbubble Destruction (UTMD) technology has been widely studied. Because the inertial cavitation occurs upon rupture of a microbubble under a high-energy ultrasonic wave, the microbubble loaded drug blasts at the target area and releases the drug locally, thereby exhibiting the therapeutic effect of targeted drug delivery. Based on the UTMD technology, the microbubble ultrasound contrast agent loaded with a magnetic particle has been studied in the prior art in order to further achieve the drug-enriched delivery and the visual controlled release. However, the stability (cavitation threshold) of the magnetic microbubble ultrasound contrast agent currently used in the UTMD technology is not ideal, and it is hard to meet requirements for in vivo cyclic targeted delivery.

Ching-Hsiang FAN et al has disclosed a magnetic microbubble ultrasound contrast agent in a paper of "Ultrasound/Magnetic Targeting with SPIODOX-Microbubble Complex for Image-Guided Drug Delivery in Brain Tumors" (THERANOSTICS, vol. 6, no. 10, 18 June 2016 (2016-06-18), pages 1542-1556, XP055756073,AU ISSN: 1838-7640, DOI: 10.7150/thno.15297), but is silent on the presence of the citric acid-based compound.

Therefore, it is of great significance to study a magnetic microbubble ultrasound contrast agent with ideal stability for treating diseases, especially for treating cancers.

### BRIEF DISCRIPTION OF THE INVENTION

The invention is as defined in claims 1 to 15.

The object of the present invention is to overcome the problem that the existing magnetic microbubble ultrasound contrast agent has poor stability, and to provide a composition for magnetic ultrasound contrast agent, a magnetic ultrasound contrast agent including the composition for magnetic ultrasound contrast agent, a preparation method of a magnetic microbubble ultrasound contrast agent, and a magnetic microbubble ultrasound contrast agent prepared by the preparation method.

The magnetic microbubble ultrasound contrast agent obtained from the composition for magnetic ultrasound contrast agent has higher stability and may meet requirements for in vivo cyclic targeted delivery, thereby achieving the local high-concentration targeted delivery of a medication.

The inventors of the present invention have found that by introducing the surfactant with specific component and ratio into a magnetic ultrasound contrast agent, the cavitation threshold of a magnetic microbubble ultrasound contrast agent may be improved effectively. The inventors of the present invention also found that, in a particularly preferred embodiment, by selecting poloxamer and sodium citrate and combining poloxamer and sodium citrate specially, the surface tension of a magnetic microbubble may be changed and the holes on the surface of the microbubble may be filled, so that the cavitation threshold and stability of the magnetic microbubble ultrasound contrast agent are improved further.

In order to achieve the above object, a first aspect of the present invention provides a composition for magnetic ultrasound contrast agent, which includes a lipid, a surfactant and a magnetic nanoparticle, wherein the surfactant consists of a surfactant A and a surfactant B, the surfactant A is in a free state, and the surfactant B is in a bonding state and is modified on the surface of the magnetic nanoparticle; the surfactant A is a nonionic surfactant whose HBL value is greater than 8, and the surfactant B is a citric acid-based compound; and relative to 1mol of the lipid, the content of the surfactant A is 0.1mol to 1mol (e.g., 0.1, 0.2, 0.3, 0.4, 0.5, 0.6, 0.7, 0.8, 0.9 or 1), the content of the surfactant B is 0.1mol to 1mol (e.g., 0.1, 0.2, 0.3, 0.4, 0.5, 0.6, 0.7, 0.8, 0.9 or 1), and the content of the magnetic nanoparticle is 0.05mol to 0.5mol (e.g., 0.05, 0.06, 0.07, 0.08, 0.09, 0.1, 0.2, 0.3, 0.4 or 0.5).

Preferably, the surfactant A is a nonionic surfactant with a HBL value is greater than 15; more preferably, a nonionic surfactant with a HBL value is 20 to 35 (e.g., 20, 21, 22, 23, 24, 25, 26, 27, 28, 29, 30, 31, 32, 33, 34 or 35).

In the present invention, the HLB value is determined by inverse gas chromatography, referring to Jiang Chaoxue, et al., Determination of Hydrophilic-lipophilic Balance of Nonionic Emulsifier by Inverse Gas Chromatography, China Synthetic Rubber Industry, 1991, 14(6): 399-401.

According to a specific embodiment, the surfactant A is any one or more selected from the group consisting of: poloxamer, poly(isobutene-maleic anhydride) (not explicitly claimed), and poly(maleic anhydride-alt-1-octadecene) (not explicitly claimed); more preferably, the surfactant A is poloxamer.

Preferably, the surfactant B is citrate and/or citrate ester.

According to a specific embodiment of the present invention, the surfactant A is poloxamer, and the surfactant B is citrate.

The ratio of the above lipid, surfactant A, surfactant B and magnetic nanoparticle according to the present invention may achieve better effects. In order to further improve stability, preferably, relative to 1mol of the lipid, the content of the surfactant A is 0.2mol to 0.6mol, the content of the surfactant B is 0.2mol to 0.5mol, and the content of the magnetic nanoparticle is 0.1mol to 0.3mol; more preferably, relative to 1mol of the lipid, the content of the surfactant A is 0.3mol to 0.5mol, the content of the surfactant B is 0.3mol to 0.4mol, and the content of the magnetic nanoparticle is 0.18mol to 0.25mol.

Preferably, the molar ratio of the magnetic particle to the surfactant B is 1:0.6 to 1:2.5, more preferably 1:1.2 to 1:1.8. The 1:1.2 to 1:1.8 is only a specific embodiment, and the upper limit may reach the saturation capacity of the surfactant B in the magnetic nanoparticle. Similarly, the upper limit 1:2.5 in the molar ratio 1:0.6 to 1:2.5 is only an illustration. In different situations, all values may be taken in the range of 1:0.6 to 1:"the saturation capacity" according to the actual situation when the saturation capacity is lower than 2.5 or higher than 2.5.

Understandably, although the magnetic microbubble ultrasound contrast agent of the present invention may be used as a drug delivery agent, the composition for ultrasound contrast agent of the present invention may not include the a drug based on the needs of production and transportation according to a specific embodiment.

According to another specific embodiment of the present invention, the composition for ultrasound contrast agent further includes a drug, relative to 100 parts by weight of a sum of the lipid, the surfactant, and the magnetic nanoparticle, the content of the drug is 1 to 20 parts by weight, more preferably 2 to 8 parts by weight.

In the present invention, the lipid may be a lipid commonly used in the art for the ultrasound contrast agent. Preferably, the lipid is phospholipids. In order to have a better synergistic effect with other components in the composition for the ultrasound contrast agent of the present invention, preferably, the lipid is any one or more selected from the group consisting of: polysorbate-based compounds and sorbitan monostearate.

In the present invention, preferably the lipid is a combination of two or more substances. According to a preferred embodiment, the lipid consists of sorbitan monostearate and polysorbate 80 in a molar ratio of 1:0.5 to 1:2 (more preferably 1:0.8 to 1:1.2).

In the present invention, the magnetic nanoparticle may be a magnetic nanoparticle commonly used in the art for the magnetic ultrasound contrast agent. Preferably, the magnetic nanoparticle is a superparamagnetic Fe₃O₄ nanoparticle.

In the present invention, preferably, the particle size of the magnetic nanoparticle is 3nm to 20nm, more preferably 7nm to 10nm.

In the present invention, the term "particle size" refers to the geometric spherical diameter of a single particle rather than an average value; when it is a range value, it means that the particle sizes of the particles in the same material fall within the range; meanwhile, the present invention allows a certain amount of error, i.e., when the particle sizes of less than 5% of the total number are not within the required range, it is also considered to meet the requirements. The particle size of the magnetic nanoparticle according to the present invention is measured by transmission electron microscope, and the particle size of the microbubble is measured by optical microscope.

In the present invention, the drug may be various drugs as required for actual treatment. For example, the drug includes: paclitaxel, adriamycin, bleomycin. All drugs used in ultrasound contrast agents in the art may be used in the present invention, and those skilled in the art may select one as required.

The composition for magnetic ultrasound contrast agent according to the present invention may also include other conventional adjuvants in the art; as long as the performance of other components is not adversely affected, those skilled in the art may choose these other adjuvants and the contents of them may refer to the conventional contents in the art.

The second aspect of the present invention provides a magnetic ultrasound contrast agent, which including the composition for magnetic ultrasound contrast agent according to the first aspect of the present invention.

According to a specific embodiment of the present invention, the ultrasound contrast agent includes a large number of gas-filled microbubbles.

In this specific embodiment, preferably, the particle size of the gas-filled microbubbles is 0.3µm to 5µm, more preferably 0.8µm to 3µm.

Preferably, the gas in the gas-filled microbubbles is an inert gas and may be commonly used in the art for microbubble ultrasound contrast agent, such as, any one or more selected from the group consisting of: perfluoropropane, perfluorobutane and sulfur hexafluoride. The shell of the gas-filled microbubbles includes the composition for ultrasound contrast agent according to the first aspect of the present invention.

According to another specific embodiment of the present invention, the ultrasound contrast agent may not contain gas-filled microbubbles. Generally in the art, the ultrasound contrast agent in this state is called film-forming agent for ultrasound contrast agent. The ultrasound contrast agent that includes a large number of gas-filled microbubbles and may be used in clinical application is obtained by the film-forming agent for ultrasound contrast agent through putting a degree of mechanical force (such as ultrasonic cavitation).

In the present invention, the term "ultrasound contrast agent" also includes film-forming agent for ultrasound contrast agent.

According to a specific embodiment of the present invention, the ultrasound contrast agent consists of a continuous phase and a dispersed phase. The continuous phase may be a conventional continuous phase used to prepare an ultrasound contrast agent in the field, such as phosphate (PBS) buffer solution. The dispersed phase includes the composition for magnetic ultrasound contrast agent according to the first aspect of the present invention and may be the gas-filled microbubbles (i.e. forming an ultrasound contrast agent) or a lipid-droplet (i.e. forming a film-forming solution for ultrasound contrast agent) .

A third aspect of the present invention provides a method for preparing a magnetic microbubble ultrasound contrast agent, wherein the raw materials include the composition for magnetic ultrasound contrast agent according to the first aspect of the present invention, and the method includes the following steps:
(1) making a first contact between the lipid and the surfactant A;
(2) mixing the drug with the resulting material obtained by step (1), pumping gases into the mixed resulting material, and processing ultrasonic cavitation to form a first microbubble suspension;
(3) processing a treatment for loading a positive charge on the first microbubble suspension to obtain a second microbubble suspension with a positive charge on the surface;
(4) making a second contact between the magnetic nanoparticle moified with the surfactant B on the surface and the obtained second microbubble suspension with a positive charge on the surface.

In the method of the third aspect of the present invention, the specific selection and ratio of the raw materials used are all processed according to the definition in the composition for magnetic ultrasound contrast agent described in the first aspect of the present invention, so that won't be covered again here.

In step (1), the first contact makes the mixing of the lipid and the surfactant A fuller, so that the core-shell structure of the microbubble in the first microbubble suspension obtained by step (2) is more stable; preferably, the first contact conditions include: a temperature of 110°C to 130°C, and a time of 8min to 16min.

In step (2), preferably, the mixing is processed at a temperature of 30°C to 50°C. For example, cooling the resulting material obtained by step (1) until the temperature fall within the range, and then processing the mixing with the drug.

In step (2), preferably, the ultrasonic cavitation conditions include: a ultrasonic power of 8kW to 12kW, and a time of 1min to 8min; more preferably, the ultrasonic cavitation conditions include: a ultrasonic power of 9kW to 11kW, and a time of 2min to 4min.

In step (3), preferably, before the treatment of loading a positive charge, leaving the first microbubble suspension to stand and centrifuge for separation to remove the microbubble with larger particle size in the upper layer, preferably, the particle size of the microbubble in the first microbubble suspension after centrifugalization is 0.3µm to 5µm, more preferably 0.8µm to 3µm.

In step (3), preferably, the process of the treatment for loading a positive charge includes: contacting the first microbubble suspension with a aqueous solution of cationic reagent.

Preferably, the volume ratio of the aqueous solution of cationic reagent and the first microbubble suspension is 0.8:1 to 1.2:1. Preferably, the concentration of the cation in the aqueous solution of the cationic reagent is 0.5mg/L to 2mg/L (e.g., 0.5, 0.6, 0.7, 0.8, 0.9, 1, 1.1, 1.2, 1.3, 1.4, 1.5, 1.6, 1.7, 1.8, 1.9 or 2).

The contacting mode between the first microbubble suspension and the aqueous solution of cationic reagent is not particularly limited, preferably, the contacting mode is ultrasonic oscillation, the ultrasonic power is 170W to 420W, and the time is 20min to 40min. The positive charge carried by the cationic reagent is adsorbed on the surface of the microbubble in the first microbubble suspension by the contacting.

In step (3), preferably, the method further includes: leaving the resulting material which contacted with the cationic reagent to stand for stratification, taking upper layer materials, and washing the upper layer materials with buffer solution, to obtained the second microbubble suspension with a positive charge on the surface.

The upper layer materials obtained after standing and stratification is an oily solution including the first microbubble suspension, and the lower layer material is an aqueous solution. Taking the upper layer solution and then washing the upper layer solution with buffer solution for 2 to 5 times to remove excess cationic reagent. The buffer solution may be commonly used in the art for acid-base buffer solution, such as phosphate (PBS) buffer solution.

In step (4), preferably, the second contact conditions include: processing the material with ultrasonic oscillation, a ultrasonic power of 170W to 420W, and a time of 15min to 50min, more preferably, the ultrasonic power of 250W to 350W, and a time for 20min to 40min.

In the present invention, preferably, the magnetic nanoparticle is a superparamagnetic nanoparticle.

In the present invention, the magnetic nanoparticle moified with the surfactant B on the surface may be prepared according to a conventional method in the art. Preferably, the preparation method of the magnetic nanoparticle moified with the surfactant B on the surface includes the following steps: ultrasonically oscillating the mixture of the magnetic nanoparticle and the aqueous solution of the surfactant B with a ultrasonic power of 170W to 420W and a time of 8min to 30min, and then separating a solid magnetic nanoparticle; this process is repeated for 2 to 5 times; thereby obtaining the magnetic nanoparticle moified with the surfactant B on the surface.

The amount of the magnetic nanoparticle and the surfactant B magnetic nanoparticle is such that the weight ratio satisfies the definition in the composition for magnetic ultrasound contrast agent in the first aspect of the present invention.

In the present invention, preferably, the magnetic nanoparticle is a superparamagnetic nanoparticle, which may be obtained by commerce or preparation. When the magnetic nanoparticle is obtained by preparation, the preparation method preferably includes: processing a thermal decomposition reaction with the mixture of ferric precursor and solvent under the water-free and oxygen-free conditions to obtain a superparamagnetic nanoparticle; and then purifying the superparamagnetic nanoparticle by magnetism.

More preferably, the preparation method preferably includes: processing a thermal decomposition reaction with the mixture of ferric acetylacetonate and triethylene glycol in a molar ratio of 2.5:1 to 3.5:1 under the water-free and oxygen-free conditions, wherein the temperature of the thermal decomposition reaction is 270°C to 290°C and the time is 0.8h to 1.2h, to obtain a superparamagnetic nanoparticle, and then purifying the superparamagnetic nanoparticle by magnetism.

Through the above technical solutions, compared with the prior art the present invention has at least the following advantages: the microbubble ultrasound contrast agent obtained by the composition for ultrasound contrast agent of the present invention may achieve the enrichment and response release of a drug at a specific site by controlling the external magnetic field and ensure sufficient stability and maintain stability in a local high flow rate state, as well as the performance of the ultrasound contrast agent in the present invention is controllable. Therefore, the present invention enables the targeted drug delivery stable and reliable in a local high flow rate state.

The endpoints of a range and any values disclosed herein are not limited to the precise ranges or values, which are to be understood to encompass values proximate to those ranges or values. For value ranges, the endpoints of each range, an endpoint of each range and an individual point value, and the individual point values may be combined with each other to yield one or more new value ranges which should be considered as particularly disclosed herein.

### BRIEF DESCRIPTION OF DRAWINGS

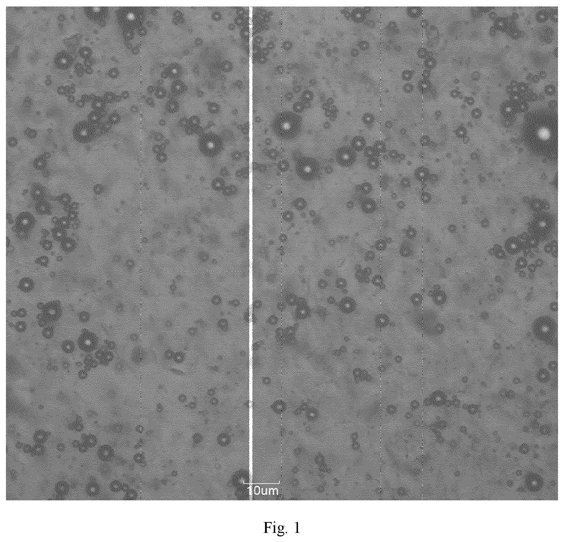

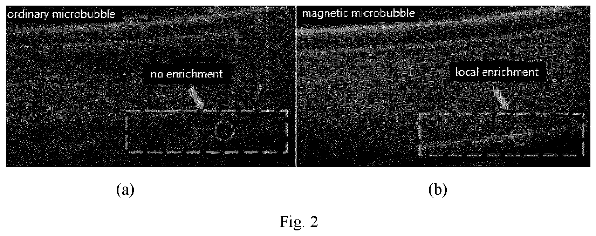

Fig. 1 is an image of the ultrasound contrast agent S1 obtained in Example 1 under an optical microscope;
(a) of Fig. 2 is an ultrasonic image of the enriched gray scale of the microbubble ultrasound contrast agent D4 obtained in Comparative Example 4;
(b) of Fig. 2 is an ultrasonic image of the enriched gray scale of the microbubble ultrasound contrast agent S1 obtained in Example 1.

### DETAILED DESCRIPTION OF THE INVENTION

The present invention will be described in detail below by the Examples. The described Examples of the present invention are only a part of the examples of the present invention, but not all of the examples.

Unless otherwise specified, the materials used in the following Examples are all commercially available analytical grades.

The following preparation examples are used to prepare the magnetic nanoparticle moified with the surfactant B on the surface using in the Examples. The following preparation example is only a specific embodiment of the present invention and not a limitation of the present invention.

### Preparation Example 1

(a) Ttriethylene glycol and ferric acetylacetonate are mixed in a molar ratio of 3:1 and heated at high temperature of 280 °C for 1h, thereby obtaining a superparamagnetic Fe₃O₄ nanoparticle. And then the particle size is observed as 7nm to 10nm by a transmission electron microscope.
(b) The superparamagnetic nanoparticle is purified by magnetism.
(c) The superparamagnetic nanoparticle obtained by step (b) and the aqueous solution (sodium citric acid solution) of surfactant B are mixed in a molar ratio of 1:1.5, and then oscillated under the ultrasonic power of 300kW. This process is repeated for 3 times.

A superparamagnetic Fe₃O₄ nanoparticle modified with the sodium citric acid on the surface is obtained.

### Preparation Example 2

Referring to the method of Preparation Example 1, the difference is that the amount of the sodium citric acid is changed so that the molar ratio of the superparamagnetic nanoparticle to the surfactant B is 1:1.8.

### Preparation Example 3

Referring to the method of Preparation Example 1, the difference is that the amount of the sodium citric acid is changed so that the molar ratio of the superparamagnetic nanoparticle to the surfactant B is 1:1.2.

### Preparation Example 4

Referring to the method of Preparation Example 1, the difference is that the amount of the sodium citric acid is changed so that the molar ratio of the superparamagnetic nanoparticle to the surfactant B is 1:0.5.

### Comparative Preparation Example 1

Referring to the method of Preparation Example 1, the difference is that the sodium citric acid is replaced with the same molar weight of poloxamer, thereby obtaining a superparamagnetic Fe₃O₄ nanoparticle modified with the poloxamer on the surface.

### Example 1

### Processed by the following steps:

(1) Sorbitan monostearate (lipid), polysorbate 80 (lipid) and poloxamer F68 (Germany BASF, P21489-25G, surfactant A, HLB value =29) are mixed well in a molar ratio of 1:1:1.5, 10mL of the obtained mixture is taken and put into a 120°C sterilization pot under the high-temperature and high-pressure conditions for 12min, and then continuously stirred and cooled until the temperature is 40°C;
(2) The resulting material obtained by step (1) and paclitaxel (chemotherapy drug) are mixed in a weight ratio of 100:5, applying ultrasonic cavitation method, perfluorocarbon gases are pumped into the obtained mixture at room temperature, processing ultrasonic cavitation for 3min under the power of 10kW, and cooling down to room temperature to obtain a first microbubble suspension;
(3) The first microbubble suspension is stood for 12h, and then centrifuged to remove the upper layer microbubbles with bigger particle size, measuring the particle size distribution of the microbubbles is in the range of 0.8µm to 3µm by a light microscope, and then 10mL polyethyleneimine solution (concentration of 1mg/L) is added slowly into the obtained microbubble suspension, oscillating under the ultrasonic power of 300kW, after allowing to adsorb for 30min, put into the 4°C refrigerator to stand for stratification, discarding the subnatant, and then the upper layer solution is washed with PBS buffer repetitive for 3 times to remove the excess polyelectrolyte, thereby obtaining the second microbubble suspension with a positive charge on the surface.
(4) 10mL of the aqueous solution of superparamagnetic nanoparticle modified with the sodium citric acid (wherein the molar ratio of the superparamagnetic nanoparticle to the lipid in the second microbubble suspension is 0.25:1) obtained by the Preparation Example 1 is added into the second microbubble suspension slowly, oscillating under the ultrasonic power of 300kW, after allowing to adsorb for 30min, put into the 4°C refrigerator to stand for stratification, discarding the subnatant, and then the upper layer solution is washed with PBS buffer repetitive for 3 times to obtain a magnetic microbubble ultrasound contrast agent, denoted as S1.

The obtained magnetic microbubble ultrasound contrast agent S1 is observed by an optical microscope and the result is shown in Figure 1. It may be seen from the Figure 1 that the contrast agent is covered in dense microbubbles with particle sizes of about 1µm, and the particle size distribution of the microbubbles is narrow, and there are no obvious impurities in the solution, which can meet the imaging requirement for the ultrasound contrast agent.

### Example 2

### Processed by the following steps:

(1) Sorbitan monostearate (lipid), polysorbate 80 (lipid) and poloxamer F127 (Sigma-Aldrich P2443-250G, surfactant A, HLB value =23) are mixed well in a molar ratio of 1:0.8:1.35, 10mL of the obtained mixture is taken and put into a 110°C sterilization pot under the high-temperature and high-pressure conditions for 16min, and then continuously stirred and cooled until the temperature is 40°C;
(2) The resulting material obtained by step (1) and paclitaxel (chemotherapy drug) are mixed in a weight ratio of 100:8, applying ultrasonic cavitation method, perfluorocarbon gases are pumped into the obtained mixture at room temperature, processing ultrasonic cavitation for 3min under the power of 10kW, and cooling down to room temperature to obtain a first microbubble suspension;
(3) The first microbubble suspension is stood for 12h, and then centrifuged to remove the upper layer microbubbles with bigger particle size, measuring the particle size distribution of the microbubbles is in the range of 0.8µm to 3µm by a light microscope, and then 10mL polyethyleneimine solution (concentration of 1.5mg/L) is added slowly into the obtained microbubble suspension, oscillating under the ultrasonic power of 250kW, after allowing to adsorb for 40min, put into the 4°C refrigerator to stand for stratification, discarding the subnatant, and then the upper layer solution is washed with PBS buffer repetitive for 3 times to remove the excess polyelectrolyte, thereby obtaining the second microbubble suspension with a positive charge on the surface.
(4) 10mL of the aqueous solution of superparamagnetic nanoparticle modified with the sodium citric acid (wherein the molar ratio of the superparamagnetic nanoparticle to the lipid in the second microbubble suspension is 0.18:1) obtained by the Preparation Example 2 is added into the second microbubble suspension slowly, oscillating under the ultrasonic power of 350kW, after allowing to adsorb for 20min, put into the 4°C refrigerator to stand for stratification, discarding the subnatant, and then the upper layer solution is washed with PBS buffer repetitive for 3 times to obtain a magnetic microbubble ultrasound contrast agent, denoted as S2.

### Example 3

### Processed by the following steps:

(1) Sorbitan monostearate (lipid), polysorbate 80 (lipid) and poloxamer F68 (Germany BASF, P21489-25G, surfactant A, HLB value =29) are mixed well in a molar ratio of 1:1.2:1.65, 10mL of the obtained mixture is taken and put into a 130°C sterilization pot under the high-temperature and high-pressure conditions for 8min, and then continuously stirred and cooled until the temperature is 40°C;
(2) The resulting material obtained by step (1) and paclitaxel (chemotherapy drug) are mixed in a weight ratio of 100:2, applying ultrasonic cavitation method, perfluorocarbon gases are pumped into the obtained mixture at room temperature, processing ultrasonic cavitation for 3min under the power of 10kW, and cooling down to room temperature to obtain a first microbubble suspension;
(3) The first microbubble suspension is stood for 12h, and then centrifuged to remove the upper layer microbubbles with bigger particle size, measuring the particle size distribution of the microbubbles is in the range of 0.8µm to 3µm by a light microscope, and then 10mL polyethyleneimine solution (concentration of 0.8mg/L) is added slowly into the obtained microbubble suspension, oscillating under the ultrasonic power of 350kW, after allowing to adsorb for 20min, put into the 4°C refrigerator to stand for stratification, discarding the subnatant, and then the upper layer solution is washed with PBS buffer repetitive for 3 times to remove the excess polyelectrolyte, thereby obtaining the second microbubble suspension with a positive charge on the surface.
(4) 10mL of the aqueous solution of superparamagnetic nanoparticle modified with the sodium citric acid (wherein the molar ratio of the superparamagnetic nanoparticle to the lipid in the second microbubble suspension is 0.25:1) obtained by the Preparation Example 3 is added into the second microbubble suspension slowly, oscillating under the ultrasonic power of 250kW, after allowing to adsorb for 40min, put into the 4°C refrigerator to stand for stratification, discarding the subnatant, and then the upper layer solution is washed with PBS buffer repetitive for 3 times to obtain a magnetic microbubble ultrasound contrast agent, denoted as S3.

### Example 4

Referring to the method of Example 1, the difference is that the ratio of the two lipids is changed, specifically, the molar ratio of sorbitan monostearate to polysorbate 80 is 1:2.5.

Finally, a magnetic microbubble ultrasound contrast agent is obtained, denoted as S4.

### Example 5

Referring to the method of Example 1, the difference is that the superparamagnetic nanoparticle modified with the sodium citric acid obtained by the Preparation Example 1 used in step (4) is replaced with the same weight of the superparamagnetic nanoparticle modified with the sodium citric acid obtained by the Preparation Example 4.

Finally, a magnetic microbubble ultrasound contrast agent is obtained, denoted as S5.

### Reference Example 6

Referring to the method of Example 1, the difference is that the poloxamer is replaced with the same molar weight of poly(isobutene-maleic anhydride) (Sigma-Aldrich, 531278-250G).

Finally, a magnetic microbubble ultrasound contrast agent is obtained, denoted as S6.

### Reference Example 7

Referring to the method of Example 1, the difference is that the poloxamer is replaced with the same molar weight of poly(maleic anhydride-alt-1-octadecene) (Sigma-Aldrich, 419117-250G).

Finally, a magnetic microbubble ultrasound contrast agent is obtained, denoted as S7.

### Example 8

Referring to the method of Example 1, the difference is that the dosage of the poloxamer is changed, specifically,.sorbitan monostearate , polysorbate 80 and poloxamer F68 are mixed in the molar ratio of 1:1:1.2.

Finally, a magnetic microbubble ultrasound contrast agent is obtained, denoted as S8.

### Example 9

Referring to the method of Example 1, the difference is that the dosage of the poloxamer is changed, specifically,.sorbitan monostearate , polysorbate 80 and poloxamer F68 are mixed in the molar ratio of 1:1:1.0.

Finally, a magnetic microbubble ultrasound contrast agent is obtained, denoted as S9.

### Example 10

Referring to the method of Example 1, the difference is that the dosage of the poloxamer is changed, specifically,.sorbitan monostearate , polysorbate 80 and poloxamer F68 are mixed in the molar ratio of 1:1:0.8.

Finally, a magnetic microbubble ultrasound contrast agent is obtained, denoted as S10.

### Example 11

Referring to the method of Example 1, the difference is that the dosage of the poloxamer is changed, specifically,.sorbitan monostearate , polysorbate 80 and poloxamer F68 are mixed in the molar ratio of 1:1:0.6.

Finally, a magnetic microbubble ultrasound contrast agent is obtained, denoted as S11.

### Example 12

Referring to the method of Example 1, the difference is that the dosage of the poloxamer is changed, specifically,.sorbitan monostearate , polysorbate 80 and poloxamer F68 are mixed well in the molar ratio of 1:1:2.

Finally, a magnetic microbubble ultrasound contrast agent is obtained, denoted as S12.

### Example 13

Referring to the method of Example 1, the difference is that the poloxamer is replaced with the same ratio weight of poloxamer P123 (Sigma-Aldrich, 435465-250ML, surfactant A, HLB value =9).

Finally, a magnetic microbubble ultrasound contrast agent is obtained, denoted as S13.

### Comparative Example 1

Referring to the method of Example 1, the difference is that no poloxamer is added.

Finally, a magnetic microbubble ultrasound contrast agent is obtained, denoted as D1.

### Comparative Example 2

Referring to the method of Example 1, the difference is that the surfactant A poloxamer used in the Example 1 is replaced with glycerol monostearate (TianWeiTaiDa RH30299-100g).

Finally, a magnetic microbubble ultrasound contrast agent is obtained, denoted as D2.

### Comparative Example 3

Referring to the method of Example 1, the difference is that the superparamagnetic nanoparticle modified with the sodium citric acid obtained by the Preparation Example 1 is replaced with the same weight of superparamagnetic Fe₃O₄ nanoparticle modified with poloxamer on the surface obtained by the Comparative Preparation Example 1.

Finally, a magnetic microbubble ultrasound contrast agent is obtained, denoted as D3.

### Comparative Example 4

Referring to the method of Example 1, the difference is that no superparamagnetic nanoparticle modified with the sodium citric acid is added, i.e., no step (3) or step (4) is performed, the first microbubble suspension with a positive charge on the surface obtained by step (2) is the final microbubble ultrasound contrast agent, denoted as D4.

### Test Example

### (1) Stability test

The stability of the ultrasound contrast agent in vivo is reflected by the half-life of the ultrasound contrast agent, and the longer the half-life, the higher the stability. Particularly, the test method includes (taking a rabbit as the object):
Selecting a length of vascular area in the ultrasonic image randomly after the ultrasound contrast agent is injected under the record of real-time ultrasound imaging, and recording the change of the average grey value in the area. Recording the point-in-time as t1 when the average grey value reaches a maximum A, and recording the point-in-time as t2 when the average grey value drops to 50% of the A, so the half-life of this kind of microbubbles is |t1-t2|. Note: Ensuring the concentration and dosage of the ultrasound contrast agent are same for each injection.

The measured half-life results of the ultrasound contrast agents from the Examples and Comparative Examples are recorded in Table 1, respectively.

### (2) Mechanical index test

In vitro enriched blasting experiment is performed. Particularly, the test method includes:
Injecting the ultrasound contrast agent of the Examples and Comparative Examples with the same concentration (10⁶pcs/mL) into the cellulose hose (inner diameter is 1mm) with ndfeb magnet (5000Gs) placed on one side respectively, and performing an in-vitro enriched blasting experiment of magnetic microbubbles under the condition of physiological flow rate (100mL/h). The ultrasonic imaging/blasting probe uses a linear array probe with 196 array elements, a center frequency of 12MHz and a bandwidth of 6MHz. Controlling the mechanical index (control range is MI=0.1-2.5, stepping is 0.1) of the ultrasonic wave emitted by the ultrasonic probe, and observing the blast of the microbubbles under different mechanical index and recording the mechanical indexes respectively when the blasting rates of the ultrasound contrast agent according to each Examples and Comparative Example are greater than 90% (time is less than 10s) (blasting rate = |before blasting - after blasting| / before blasting*100%), and the results are recorded in Table 1.

### (3) Magnetism test

In vitro enrichment experiment is performed. Particularly, the test method includes:
Injecting the ultrasound contrast agent of the Examples and Comparative Examples with the same concentration (10⁶pcs/mL) into the cellulose hose (inner diameter is 1mm) with ndfeb magnet (5000Gs) placed on one side respectively, and performing an in-vitro enriched blasting experiment of magnetic microbubbles under the condition of static flow rate (0mL/h). The ultrasonic imaging probe uses a linear array probe with 196 array elements, a center frequency of 12MHz and a bandwidth of 6MHz. Controlling the mechanical index (control range is MI<0.1) of the ultrasonic wave emitted by the ultrasonic probe, and observing the magnetism of the magnetic microbubbles under different mechanical index.
Within 15min, if almost all the magnetic microbubbles are enriched on the magnet side (ultrasonic image shows that the gray characteristic of the magnetic microbubbles on the magnet side is larger than 95% of all the microbubbles signal), it is considered that the magnetism is normal;
Within 15min, if more than half of the magnetic microbubbles are enriched on the magnet side (ultrasonic image shows that the gray characteristic of the magnetic microbubbles on the magnet side is larger than 50% of all the microbubbles signal), it is considered that the magnetism is weak;
Within 15min, if the magnetic microbubbles are uniformly distributed and have no adsorption effect (ultrasonic image shows that the gray characteristic of the magnetic microbubbles on the magnet side is distributed in the cellulose hose uniformly), it is considered as no magnetism.

(4) Taking the magnetic microbubble ultrasound contrast agent S1 of Example 1 and the ultrasound contrast agent D4 of Comparative Example 4 as examples, after 10 enriched blasting experiment, the enriched gray scales of S1 and D4 are compared, as shown in (a) of Fig. 2 and (b) of Fig. 2. It can be significantly seen from Figure 2 that S1 has an obvious local enrichment while D4 has no enrichment.

**Table 1:**

| | Half-life (min) | Blasting mechanical index MI | Magnetism |
|---|---|---|---|
| S1 | 6.3 | 1.1 | normal |
| S2 | 5.8 | 1.1 | normal |
| S3 | 5.6 | 1.0 | normal |
| S4 | 5.3 | 0.9 | normal |
| S5 | 5.6 | 1.0 | weak |
| S6 | 5.1 | 0.8 | normal |
| S7 | 5.0 | 0.9 | normal |
| S8 | 6.2 | 1 | normal |
| S9 | 6 | 0.9 | normal |
| S10 | 5.6 | 0.9 | normal |
| S11 | 5.5 | 0.8 | normal |
| S12 | 5.5 | 0.8 | normal |
| S13 | 5.0 | 0.8 | normal |
| D1 | 2 | 0.3 | normal |
| D2 | 2.5 | 0.3 | normal |
| D3 | 6.5 | 1.1 | no magnetism |
| D4 | 6.8 | 1.2 | no magnetism |

It can be seen from Table 1, the composition for ultrasound contrast agent and the ultrasound contrast agent according to the present invention have both longer half-life and higher blasting mechanical index, as well as good magnetism, thereby the requirements for in vivo cyclic targeted delivery may be met by the magnetic microbubble ultrasound contrast agent.

And it can be seen from Example 1, Example 8 to Example 11, when the amount of emulsifier is in a proper range, the higher the content of emulsifier, the longer half-life of the magnetic ultrasound contrast agent and the higher threshold of blasting mechanical index; It can be seen from Example 12, when the content of the emulsifier is too high, the half-life of the magnetic ultrasound contrast agent and the threshold of blasting mechanical index are decreased. Therefore, the performance of the ultrasound contrast agent is controllable and is of great significance for practical application.

## Claims

1. A composition for magnetic ultrasound contrast agent, **characterized by** comprising a lipid, a surfactant and a magnetic nanoparticle; wherein:
the surfactant consists of a surfactant A and a surfactant B, wherein the surfactant A is in a free state, and the surfactant B is modified on the surface of the magnetic nanoparticle;
the surfactant A is a nonionic surfactant whose HLB value is greater than 8 and is poloxamer;
the surfactant B is citrate and/or citrate ester; and
relative to 1mol of the lipid, a content of the surfactant A is 0.1mol to 1mol, a content of the surfactant B is 0.1mol to 1mol, and a content of the magnetic nanoparticle is 0.05mol to 0.5mol.

2. The composition for magnetic ultrasound contrast agent according to claim 1, **characterized in that** the surfactant A is a nonionic surfactant whose HLB value is greater than 15; preferably, the surfactant A is a nonionic surfactant whose HLB value is 20 to 35;
preferably, the surfactant A is poloxamer and the surfactant B is citrate.

3. The composition for magnetic ultrasound contrast agent according to claim 1 or 2, **characterized in that** relative to 1mol of the lipid, the content of the surfactant A is 0.2mol to 0.6mol, the content of the surfactant B is 0.2mol to 0.5mol, and the content of the magnetic nanoparticle is 0.1mol to 0.3mol.

4. The composition for magnetic ultrasound contrast agent according to any one of claims 1 to 3, **characterized in that** the molar ratio of the magnetic particle to the surfactant B is 1:0.6 to 1:2.5.

5. The composition for magnetic ultrasound contrast agent according to any one of claims 1 to 4, **characterized by** further comprising a drug, and relative to 100 parts by weight of a sum of the lipid, the surfactant and the magnetic nanoparticle, the content of the drug is 1 to 20 parts by weight.

6. The composition for magnetic ultrasound contrast agent according to any one of claims 1 to 5, **characterized in that** the lipid consists of sorbitan monostearate and polysorbate 80 in a molar ratio of 1:0.5 to 1:2.

7. The composition for magnetic ultrasound contrast agent according to any one of claims 1 to 6, **characterized in that** the magnetic nanoparticle is a superparamagnetic Fe₃O₄ nanoparticle with a particle size of 3nm to 20nm.

8. A magnetic ultrasound contrast agent, **characterized in that** the magnetic ultrasound contrast agent comprises, or is prepared from, the composition for magnetic ultrasound contrast agent according to any one of claims 1 to 7.

9. A method for preparing a magnetic microbubble ultrasound contrast agent, **characterized in that** the raw materials comprise the composition for magnetic ultrasound contrast agent according to any one of claims 1 to 7, wherein the method comprises the following steps:
(1) making a first contact between the lipid and the surfactant A;
(2) mixing the drug with the resulting material obtained by step (1), pumping gases into the mixed resulting material, and processing ultrasonic cavitation to form a first microbubble suspension;
(3) processing a treatment for loading a positive charge on the first microbubble suspension to obtain a second microbubble suspension with a positive charge on the surface;
(4) making a second contact between the magnetic nanoparticle moified with the surfactant B on the surface and the obtained second microbubble suspension with a positive charge on the surface.

10. The method according to claim 9, **characterized in that** the first contact conditions comprise: a temperature of 110°C to 130°C, and a time of 8min to 16min.

11. The method according to claim 9 or 10, **characterized in that** in step (2), the mixing is processed at a temperature of 30°C to 50°C.

12. The method according to any one of claims 9 to 11, **characterized in that** the ultrasonic cavitation conditions comprise: an ultrasonic power of 8kW to 12kW, and a time of 1min to 8min.

13. The method according to any one of claims 9 to 12, **characterized in that** in step (3), the process of the treatment for loading a positive charge comprises: contacting the first microbubble suspension with an aqueous solution of cationic reagent;
preferably, the volume ratio of the aqueous solution of cationic reagent and the first microbubble suspension is 0.8:1 to 1.2:1, and the concentration of the cation in the aqueous solution of the cationic reagent is 0.5mg/L to 2mg/L.

14. The method according to any one of claim 9 to 13, **characterized by** further comprising: leaving the resulting material, which have contacted with the cationic reagent, to stand for stratification, taking upper layer materials, and washing the upper layer materials with buffer solution.

15. The method according to any one of claims 9 to 14, **characterized in that** in step (4), the second contact conditions comprise: processing the material with ultrasonic oscillation with an ultrasonic power of 170 W to 420W, and a time of 15 min to 50min.

## Patentansprüche

1. Eine Zusammensetzung für ein magnetisches Ultraschallkontrastmittel, **dadurch gekennzeichnet, dass** sie ein Lipid, ein Tensid und ein magnetisches Nanopartikel umfasst; wobei das Tensid aus einem Tensid A und einem Tensid B besteht, wobei das Tensid A in freiem Zustand vorliegt und das Tensid B auf der Oberfläche des magnetischen Nanopartikels modifiziert ist;
das Tensid A ein nichtionisches Tensid mit einem HLB-Wert von mehr als 8 ist und das Tensid A Poloxamer ist;
das Tensid B Citrat und/oder Citratester ist; und
bezogen auf 1 mol des Lipids, der Gehalt des Tensids A 0,1 mol bis 1 mol, der Gehalt des Tensids B 0,1 mol bis 1 mol und der Gehalt des magnetischen Nanopartikels 0,05 mol bis 0,5 mol beträgt.

2. Die Zusammensetzung für ein magnetisches Ultraschallkontrastmittel nach Anspruch 1, **dadurch gekennzeichnet, dass** das Tensid A ein nichtionisches Tensid mit einem HLB-Wert von mehr als 15 ist; vorzugsweise, wobei das Tensid A ein nichtionisches Tensid mit einem HLB-Wert von 20 bis 35 ist;
vorzugsweise, wobei das Tensid A Poloxamer ist und das Tensid B Citrat ist.

3. Die Zusammensetzung für ein magnetisches Ultraschallkontrastmittel nach Anspruch 1 oder 2, **dadurch gekennzeichnet, dass** bezogen auf 1 mol des Lipids der Gehalt des Tensids A 0,2 mol bis 0,6 mol, der Gehalt des Tensids B 0,2 mol bis 0,5 mol und der Gehalt des magnetischen Nanopartikels 0,1 mol bis 0,3 mol beträgt.

4. Die Zusammensetzung für ein magnetisches Ultraschallkontrastmittel nach einem der Ansprüche 1 bis 3, **dadurch gekennzeichnet, dass** das Molverhältnis des magnetischen Nanopartikels zum Tensid B 1:0,6 bis 1:2,5 beträgt.

5. Die Zusammensetzung für ein magnetisches Ultraschallkontrastmittel nach einem der Ansprüche 1 bis 4, **dadurch gekennzeichnet, dass** sie ferner ein Medikament umfasst, und bezogen auf 100 Gewichtsteile der Summe aus Lipid, Tensid und magnetischem Nanopartikel der Gehalt des Medikaments 1 bis 20 Gewichtsteile beträgt.

6. Die Zusammensetzung für ein magnetisches Ultraschallkontrastmittel nach einem der Ansprüche 1 bis 5, **dadurch gekennzeichnet, dass** das Lipid aus Sorbitanmonostearat und Polysorbat 80 im Molverhältnis von 1:0,5 bis 1:2 besteht.

7. Die Zusammensetzung für ein magnetisches Ultraschallkontrastmittel nach einem der Ansprüche 1 bis 6, **dadurch gekennzeichnet, dass** das magnetische Nanopartikel ein superparamagnetisches Fe₃O₄-Nanopartikel mit einer Partikelgröße von 3 nm bis 20 nm ist.

8. Ein magnetisches Ultraschallkontrastmittel, **dadurch gekennzeichnet, dass** das magnetische Ultraschallkontrastmittel die Zusammensetzung nach einem der Ansprüche 1 bis 7 enthält oder aus der Zusammensetzung nach einem der Ansprüche 1 bis 7 hergestellt ist.

9. Ein Verfahren zur Herstellung eines magnetischen Mikroblasen-Ultraschallkontrastmittels, **dadurch gekennzeichnet, dass** die Rohstoffe eine Zusammensetzung für ein magnetisches Ultraschallkontrastmittel nach einem der Ansprüche 1 bis 7 umfassen, wobei das Verfahren die folgenden Schritte umfasst:
(1) Durchführung eines ersten Kontakts zwischen dem Lipid und dem Tensid A;
(2) Mischen eines Medikaments mit dem im Schritt (1) erhaltenen Material, Einleiten von Gasen in das gemischte Material und Durchführung einer Ultraschallkavitation zur Bildung einer ersten Mikroblasensuspension;
(3) Durchführung einer Behandlung zum Beladen mit einer positiven Ladung an der ersten Mikroblasensuspension zur Gewinnung einer zweiten Mikroblasensuspension mit einer positiven Oberflächenladung;
(4) Durchführung eines zweiten Kontakts zwischen dem auf der Oberfläche mit dem Tensid B modifizierten magnetischen Nanopartikel und der zweiten Mikroblasensuspension mit einer positiven Oberflächenladung.

10. Das Verfahren nach Anspruch 9, **dadurch gekennzeichnet, dass** die Bedingungen des ersten Kontakts Folgendes umfassen: eine Temperatur von 110 °C bis 130 °C und eine Dauer von 8 min bis 16 min.

11. Das Verfahren nach Anspruch 9 oder 10, **dadurch gekennzeichnet, dass** das Mischenin Schritt (2) bei einer Temperatur von 30 °C bis 50 °C durchgeführt wird.

12. Das Verfahren nach einem der Ansprüche 9 bis 11, **dadurch gekennzeichnet, dass** die Bedingungen der Ultraschallkavitation Folgendes umfassen: eine Ultraschallleistung von 8 kW bis 12 kW und eine Dauer von 1 min bis 8 min.

13. Das Verfahren nach einem der Ansprüche 9 bis 12, **dadurch gekennzeichnet, dass** der Vorgang der Behandlung zum Beladen mit einer positiven Ladung in Schritt (3) Folgendes umfasst: Kontaktieren der ersten Mikroblasensuspension mit einer wässrigen Lösung eines kationischen Reagenzes;
vorzugsweise, wobei das Volumenverhältnis der wässrigen Lösung des kationischen Reagenzes zur ersten Mikroblasensuspension 0,8:1 bis 1,2:1 beträgt und die Konzentration des Kations in der wässrigen Lösung des kationischen Reagenzes 0,5 mg/L bis 2 mg/L beträgt.

14. Das Verfahren nach einem der Ansprüche 9 bis 13, **dadurch gekennzeichnet, dass** es ferner folgende Schritte umfasst: Stehenlassen des mit dem kationischen Reagenz in Kontakt gebrachten Materials zur Phasentrennung, Entnahme der oberen Schicht und Waschen der oberen Schicht mit einer Pufferlösung.

15. Das Verfahren nach einem der Ansprüche 9 bis 14, **dadurch gekennzeichnet, dass** die Bedingungen des zweiten Kontakts in Schritt (4) Folgendes umfassen: Behandlung des Materials mittels Ultraschalloszillation mit einer Ultraschallleistung von 170 W bis 420 W und einer Dauer von 15 min bis 50 min.

## Revendications

1. Une composition pour produit de contraste ultrasonore magnétique, **caractérisée par** le fait de comprendre un lipide, un tensioactif et une nanoparticule magnétique ; où :
le tensioactif consiste en un tensioactif A et un tensioactif B, où le tensioactif A est dans un état libre, et le tensioactif B est modifié sur la surface de la nanoparticule magnétique ;
le tensioactif A est un tensioactif non ionique dont la valeur HLB est supérieure à 8 et est un poloxamère ;
le tensioactif B est un citrate et/ou un ester de citrate ; et
par rapport à 1 mol du lipide, une teneur en tensioactif A est de 0,1 mol à 1 mol, une teneur en tensioactif B est de 0,1 mol à 1 mol, et une teneur en nanoparticule magnétique est de 0,05 mol à 0,5 mol.

2. La composition pour produit de contraste ultrasonore magnétique selon la revendication 1, **caractérisée en ce que** le tensioactif A est un tensioactif non ionique dont la valeur HLB est supérieure à 15 ; de préférence, le tensioactif A est un tensioactif non ionique dont la valeur HLB est de 20 à 35 ;
de préférence, le tensioactif A est un poloxamère et le tensioactif B est un citrate.

3. La composition pour produit de contraste ultrasonore magnétique selon la revendication 1 ou 2, **caractérisée en ce que** par rapport à 1 mol du lipide, la teneur en tensioactif A est de 0,2 mol à 0,6 mol, la teneur en tensioactif B est de 0,2 mol à 0,5 mol, et la teneur en nanoparticule magnétique est de 0,1 mol à 0,3 mol.

4. La composition pour produit de contraste ultrasonore magnétique selon l'une quelconque des revendications 1 à 3, **caractérisée en ce que** le rapport molaire de la particule magnétique au tensioactif B est de 1/0,6 à 1/2,5.

5. La composition pour produit de contraste ultrasonore magnétique selon l'une quelconque des revendications 1 à 4, **caractérisée par** le fait de comprendre en outre un médicament, et par rapport à 100 parties en poids d'une somme du lipide, du tensioactif et de la nanoparticule magnétique, la teneur en médicament est de 1 à 20 parties en poids.

6. La composition pour produit de contraste ultrasonore magnétique selon l'une quelconque des revendications 1 à 5, **caractérisée en ce que** le lipide consiste en monostéarate de sorbitane et polysorbate 80 dans un rapport molaire de 1/0,5 à 1/2.

7. La composition pour produit de contraste ultrasonore magnétique selon l'une quelconque des revendications 1 à 6, **caractérisée en ce que** la nanoparticule magnétique est une nanoparticule de Fe₃O₄ superparamagnétique avec une taille de particule de 3 nm à 20 nm.

8. Un produit de contraste ultrasonore magnétique, **caractérisé en ce que** le produit de contraste ultrasonore magnétique comprend, ou est préparé à partir de, la composition pour produit de contraste ultrasonore magnétique selon l'une quelconque des revendications 1 à 7.

9. Une méthode pour la préparation d'un produit de contraste ultrasonore à microbulles magnétiques, **caractérisée en ce que** les matières premières comprennent la composition pour produit de contraste ultrasonore magnétique selon l'une quelconque des revendications 1 à 7, où la méthode comprend les étapes suivantes :
(1) réaliser un premier contact entre le lipide et le tensioactif A ;
(2) mélanger le médicament avec la matière résultante obtenue par l'étape (1), pomper des gaz dans la matière résultante mélangée, et procéder à une cavitation ultrasonore afin de former une première suspension de microbulles ;
(3) procéder à un traitement pour le chargement d'une charge positive sur la première suspension de microbulles afin d'obtenir une deuxième suspension de microbulles avec une charge positive sur la surface ;
(4) réaliser un deuxième contact entre la nanoparticule magnétique modifiée avec le tensioactif B sur la surface et la deuxième suspension de microbulles obtenue avec une charge positive sur la surface.

10. La méthode selon la revendication 9, **caractérisée en ce que** les conditions de premier contact comprennent : une température de 110 °C à 130 °C, et un temps de 8 min à 16 min.

11. La méthode selon la revendication 9 ou 10, **caractérisée en ce que** dans l'étape (2), il est procédé au mélange à une température de 30 °C à 50 °C.

12. La méthode selon l'une quelconque des revendications 9 à 11, **caractérisée en ce que** les conditions de cavitation ultrasonore comprennent : une puissance ultrasonore de 8 kW à 12 kW, et un temps de 1 min à 8 min.

13. La méthode selon l'une quelconque des revendications 9 à 12, **caractérisée en ce que** dans l'étape (3), le procédé du traitement pour le chargement d'une charge positive comprend le fait : de mettre en contact la première suspension de microbulles avec une solution aqueuse de réactif cationique ;
de préférence, le rapport volumique de la solution aqueuse de réactif cationique et de la première suspension de microbulles est de 0,8/1 à 1,2/1, et la concentration du cation dans la solution aqueuse du réactif cationique est de 0,5 mg/L à 2 mg/L.

14. La méthode selon l'une quelconque des revendications 9 à 13, **caractérisée par** le fait de comprendre en outre le fait :
de laisser la matière résultante, qui a été mise en contact avec le réactif cationique, reposer pour stratification, de prendre des matières de couche supérieure, et de laver les matières de couche supérieure avec une solution tampon.

15. La méthode selon l'une quelconque des revendications 9 à 14, **caractérisée en ce que** dans l'étape (4), les conditions de deuxième contact comprennent le fait : de procéder sur la matière à une oscillation ultrasonore avec une puissance ultrasonore de 170 W à 420 W, et un temps de 15 min à 50 min.
